Europäisches Patentamt

European Patent Office

Office européen des brevets

(19)

(11) Veröffentlichungsnummer: **0 506 618 B1**

(12) # EUROPÄISCHE PATENTSCHRIFT

(45) Veröffentlichungstag der Patentschrift: **05.07.95**

(51) Int. Cl.[6]: **A61B 17/39**

(21) Anmeldenummer: **92810203.7**

(22) Anmeldetag: **20.03.92**

(54) **Speisegerät für bipolare Elektroden zur Kapsulotomie.**

(30) Priorität: **28.03.91 CH 965/91**

(43) Veröffentlichungstag der Anmeldung:
**30.09.92 Patentblatt 92/40**

(45) Bekanntmachung des Hinweises auf die
Patenterteilung:
**05.07.95 Patentblatt 95/27**

(84) Benannte Vertragsstaaten:
**AT CH DE ES FR GB IT LI SE**

(56) Entgegenhaltungen:
**EP-A- 0 358 336
GB-A- 2 132 893
US-A- 4 378 801
US-A- 4 481 948**

**KLIN. MBL. AUGENHEILK. 184 Nr. 5, Mai 1984,
STUTTGART Seiten 442 - 444; R.KLÖTI: 'Bipolar-Nassfeld-Diathermie in der Mikrochirurgie'**

**U.Tietze, Ch. Schenk, "Halbleiter-Schaltungstechnik", fünfte Auflage, Springer-Verlag
Berlin Heidelberg New York 1980, Seiten 405
bis 407**

**C.Paganoni, G. Leopardi, "Diathermic high-**

**frequency capsulorhexis", Ocular Surgery
News, Internationale Ausgabe, Mai 1993, Seite 42**

(73) Patentinhaber: **OERTLI-INSTRUMENTE AG
Gemperenstrasse 18
CH-9442 Berneck (CH)**

(72) Erfinder: **Oertli, Heinz A.
Schwendibühl
CH-9053 Teufen (CH)**

(74) Vertreter: **Schick, Carl et al
Isler & Pedrazzini AG
Patentanwälte
Postfach 6940
CH-8023 Zürich (CH)**

Rank Xerox (UK) Business Services
(3.10/3.09/3.3.3)

## Beschreibung

Die vorliegende Erfindung betrifft eine Kapsulotomie-Vorrichtung für die Augenmikrochirurgie gemäss dem Oberbegriff des Patentanspruchs 1.

Eine derartige Vorrichtung ist aus der Fachzeitschrift "Klinische Monatsblätter für Augenheilkunde" 184 (1984), Seiten 331 bis 512, insbesondere Seiten 442 bis 444, bekannt.

Dort ist eine Bipolar-Nassfeld-Diathermie in der Augenmikrochirurgie beschrieben, die die bisherigen Verfahren zur Oeffnung der Linsenkapsel durch Schlitzen des Kapselsacks, Oeffnen des Kapselsacks durch Perforieren und anschliessendes Reissen oder durch Capsulorhexis ersetzen.

Von den ersten Vitrektomien im Jahr 1972 ausgehend, wurde die Möglichkeit einer Hämostase im Glaskörper untersucht. Dazu wurde als geeignetes Instrument ein koaxiales Bipolar-Diathermiegerät entwickelt. Für Operationen am Auge muss aber sehr darauf geachtet werden, dass die Erwärmung nicht zu stark ist. Deshalb war damals das Gerät mit einer Aspirations-Infusions-Spülvorrichtung ausgerüstet, um austretendes flüssiges Blut zu entfernen. Auf jeden Fall wird dort bei Verwendung eines modulierten Hochfrequenzstromes auf die Notwendigkeit einer zusätzlichen Kühlung hingewiesen, ohne konkrete Massnahmen über eine optimale Modulierungsart anzugeben.

Aus der GB-A-2.132.893 ist ein Chirurgiegerät der Veterinärmedizin mit einem gepulsten Hochfrequenzgenerator bekannt, bei dem das Sperrzeit/Durchlasszeit-Verhältnis zwischen 1/3 und 3 oder die relative Einschaltdauer zwischen 25% und 75% liegt. Dieses Gerät, das mit einer Frequenz von 2 MHz arbeitet, wurde für ein relativ grossflächiges Zerstören von äusserlichen gut- oder bösartigen Tumorgeweben am Augenlied oder am Augenbulbus von Rindern entwickelt.

Die US-4,211,230 beschreibt eine Vorrichtung zur elektrochirurgischen Koagulation mit einem gepulsten Radiofrequenzgenerator im Bereich von 1,75 MHz und einem mit Hilfe eines Zufallgenerators gesteuerten Pulsbreitenmodulator, der eine durchschnittliche relative Einschaltdauer von etwa 10% ergibt.

Aus der EP-A1-0 358 336 ist ferner ein Chirurgiegerät der Humanmedizin zur Zerstörung von Geweben bekannt, das einen gepulsten Hochfrequenzgenerator für Impulsspannungen im Bereich von 500 bis 3600 Volt und Impulsbreiten im Bereich von 5 bis 500 Mikrosekunden aufweist.

Zur diathermischen Oeffnung des Kapselsackes sind zwei Instrumente bekannt geworden.

Im US-Patent Nr. 4,367,744 trägt ein elektrisch isolierendes Handstück einen Ausbrennring, der von hinten am Handstück eindrähtig mit elektrischem Strom gespeist wird. Damit ein Strom fliessen kann, wurde vorgeschlagen, zweidrähtig zum Ausbrennring zu führen. Beim Auflegen des Ausbrennrings auf den Linsensack erzeugt der Strom Hitze im Bereich von 500°C bis 2000°C. Eine derart hohe Temperatur darf nur kurzzeitig wirken und die ausgebrannte Stelle am Linsensack muss so rasch wie möglich gekühlt werden. Weil dabei der Ausbrennring schon heiss ins Auge eingeführt werden muss, um die Operation in kürzester zeit ausführen zu können, muss äusserst vorsichtig gearbeitet werden, um keine anderen Augenpartien zu beschädigen.

Das US-Patent Nr. 4,481,948 beschreibt einen gleichartigen Ausbrennring, der jedoch nicht mit im Ring fliessendem Strom geheizt wird, sondern es wird dem Ring hochfrequenter Strom mit 10 kHz zugeführt. Ein Gegenpol wird unter den Patienten gelegt, um damit den Stromkreis zu schliessen. Der hochfrequente Strom wird durch den Arzt mittels eines Pedals eingeschaltet.

Es kann auch mit dieser Anordnung eine hohe Wärme entstehen, die für das Gewebe im Auge sehr schädlich sein kann. Gemäss dem älteren Patent musste die heisse Schlinge in die vordere Augenkammer eingebracht werden, bis sie dann durch die Pupille in der hinteren Augenkammer auf den Linsensack gelegt werden kann, um dort eine Oeffnung auszubrennen.

Mit dem hochfrequenten Strom kann die Schlinge nach dem Willen des Arztes aktiviert, d.h. erwärmt werden. Der hochfrequente Strom kann auch erst dann fliessen, wenn die Schlinge auf einer mit leitender Flüssigkeit bedeckten Stelle aufgelegt wird, also auf dem Linsensack. Auch damit lässt sich die einwirkende Wärme für die Koagulation am Rand der Oeffnung nicht beherrschen, so dass leicht Schädigungen durch Hitzeeinwirkung entstehen können.

Es ist deshalb eine Aufgabe der Erfindung, die hochfrequente Stromzufuhr derart zu steuern, dass nur gerade genügend Wärme zum Aufschneiden des Linsensackes und zur Koagulation des Lochrandes entsteht, das übrige Gewebe aber vor Wärmeinwirkung leichter geschützt werden kann.

Erfindungsgemäss wird dies durch eine Kapsulotomie-Vorrichtung mit den im kennzeichnenden Teil des unabhängigen Patentanspruchs 1 angegebenen Merkmalen erreicht. Ein Vorteil der erfindungsgemässen Vorrichtung besteht darin, dass man damit eine einwandfreie Kapsulotomie unter Vermeidung von Schädigungen am Rand der Oeffnung durchführen kann, und zwar ohne Verwendung einer Abkühlungsflüssigkeit.

Weitere vorteilhafte Ausführungen der Erfindung sind in den abhängigen Ansprüchen angegeben.

Nachfolgend wird die Erfindung an einem Ausführungsbeispiel anhand der Zeichnung erläutert.

Es zeigt:

Fig. 1 ein vergrösserter Ausschnitt in Schnittansicht eines menschlichen Auges,

Fig. 2 denselben Ausschnitt in perspektivischer Ansicht mit einem bipolaren Diathermie-Instrument für eine Kapsulotomie,

Fig. 3 Blockschema einer erfindungsgemässen Vorrichtung mit einem bipolaren Diathermie-Instrument, und

Fig. 4 zwei Impulsdiagramme für den Stromfluss aus einer solchen Vorrichtung.

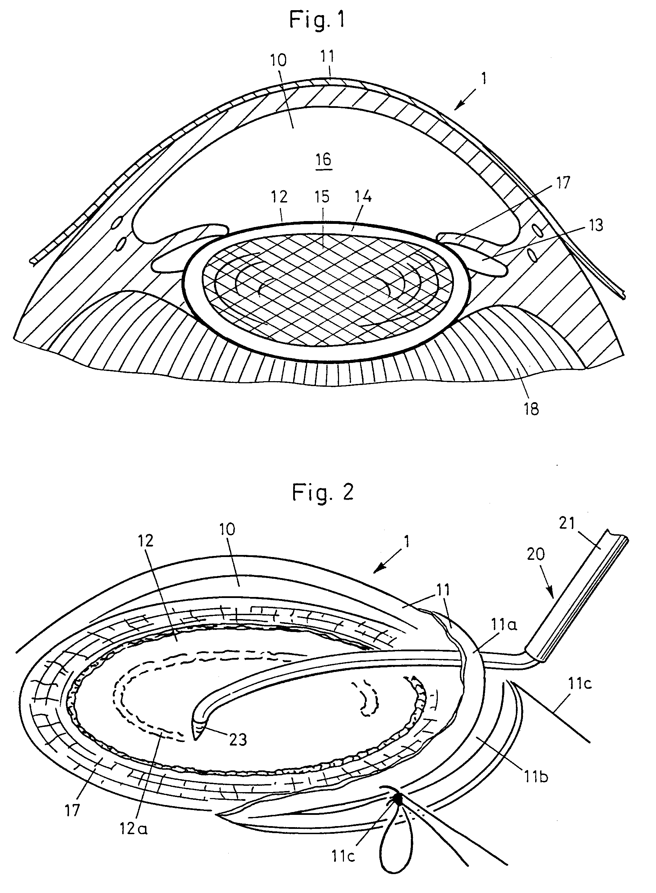

Fig. 1 zeigt einen vorderen Ausschnitt eines menschlichen Auges 1 in stark vergrössertem Massstab. Dieser Ausschnitt zeigt deutlich die vordere Augenkammer 10, die nach aussen durch die Hornhaut 11 abgeschlossen ist. Die Linsenkapsel besteht aus dem Kapselsack 12, dem Linsenkern 15 sowie der dazwischenliegenden Rindenschicht (Cortex) 14. Darüber liegt die ausgeweitete Iris 17, wobei die Pupille 16 eine grosse Oeffnung bildet. Hinter der Iris 17 befindet sich die hintere Augenkammer 13. Der Linsensack 12 mit der Linse 15 liegt zwischen der Iris 17 und der Sklera 18.

Fig. 2 zeigt eine Ansicht auf das Auge 1 nach Fig. 1 von schräg oben, so dass die ganze Iris 17 ersichtlich ist. Die Hornhaut 11 ist an ihrem Rand 11a aufgeschnitten, um einen Zugang in die vordere Augenkammer 10 zu schaffen. Der Schnitt 11b ist beidseits durch eine Schlinge 11c zusammengehalten, damit er nicht aufreissen kann. Durch diesen Schlitz wird ein bipolares Diathermie-Instrument 20 mit Handstück 21 eingeführt, aus dem die bipolare Spitze 23 herausschaut. Diese bipolare Spitze 23 ist gebogen, damit das Instrument seitlich des Auges 1 eingeführt und parallel zum Linsensack 12 zur Schnittstelle geführt werden kann, wo dann der Schnitt 12a im Linsensack 12 mit der gebogenen Spitze 23 durchgeführt wird.

Der Arzt kann nun mittels hochfrequentem Strom, der an die bipolare Spitze 23 gelangt, den Linsensack 12 koagulieren oder mit höherer elektrischer Leistung koagulieren und durchtrennen. Das Durchtrennen stösst dabei auf die eingangs erwähnte Schwierigkeit infolge starker Erhitzung des Arbeitsumfeldes.

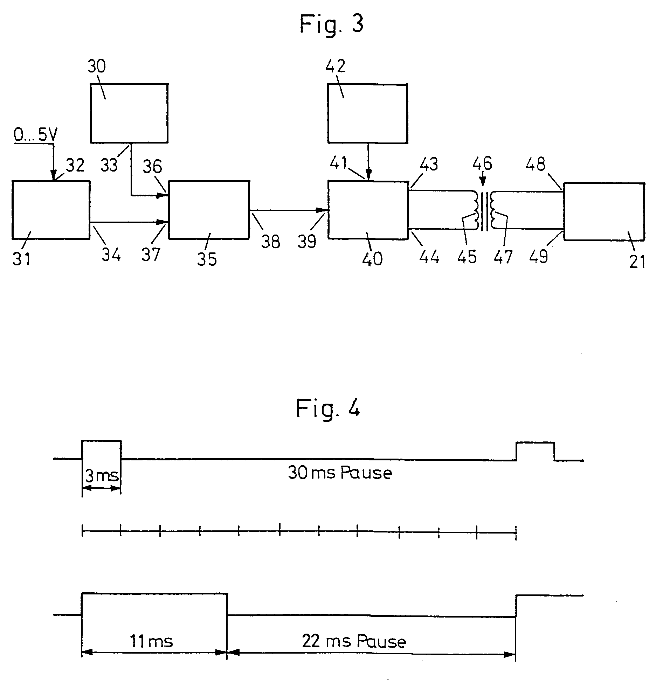

Erfindungsgemäss ist nun eine Vorrichtung (Fig. 3) vorgesehen, mit der ein pulsbreitenmodulierter Hochfrequenzstrom erzeugt werden kann. Die Vorrichtung umfasst demgemäss einen Hochfrequenzgenerator 30 und eine Pulsbreitensteuerung 31, die an einem Sollwerteingang 32 mit einer Steuerspannung von beispielsweise 0 bis +5 V gespeist wird. Der Ausgang 33 des Hochfrequenzgenerators 30 und der Ausgang 34 der Pulsbreitensteuerung 31 werden Eingängen 36 und 37 eines UND-Tores 35 zugeführt, dessen Ausgang 38 mit dem Eingang 39 eines Leistungsverstärkers 40 verbunden ist. Der Leistungsverstärker 40 wird am Eingang 41 aus einer stabilisierten Spannungsquelle 42 gespeist. Die Spannungsquelle gibt einen Strom von 3 A bei einer Spannung von 18 V ab und umfasst eine Kurzschlussüberwachung. Die Ausgänge 43, 44 sind der Primärwicklung 45 eines Uebertragers 46 zugeführt, von dessen Sekundärwicklung 47 Leitungen 48, 49 zum Handstück 21 des bipolaren Diathermie-Instrumentes 20 führen.

Durch diese Anordnung kann durch Wahl der Steuerspannung von 0 bis 5 V die hochfrequente Spannung mit einer Frequenz von beispielsweise 500 kHz getaktet werden, derart, dass bei einer Steuerspannung von 0,5 bis 5 V ein frequenzkonstantes Rechtecksignal mit einer Frequenz von 15 Hz bzw. 30 Hz und in diesem Fall beispielsweise mit einer Impulsdauer von 3 bis 11 ms und einer Impulspause von 30 bis 22 ms erzeugt wird, mit dem das UND-Tor getaktet wird. Bei höherer Steuerspannung wird der Impuls breiter und die Impulspause entsprechend kürzer (Fig. 4). Die Frequenz der Rechteckschwingung bleibt konstant. Durch die Verknüpfung im UND-Tor 35 kann der Leistungsverstärker 40 gespeist werden, der mit Hilfe der stabilisierten Spannung eine Impulsleistung von $P_{max} = 54$ W abgeben kann. Durch diese hohe Impulsleistung wird das Gewebe des Linsensackes durchtrennt, und es wird nicht nur eine Koagulation erreicht. Die impulsweise Zuführung der hochfrequenten Schwingungen mit einer Impulspause, die mindestens zweimal so lange dauert wie der Impuls, gestattet eine genügend starke Abkühlung, um das benachbarte Gewebe nicht zu gefährden. Durch den Uebertrager wird das Kapsulotomie-Handstück vom Gerät isoliert, so dass kein erdspannungsbehaftetes Potential an diesem Instrument vorhanden ist. Damit der hochfrequente Strom zwischen den Polen des bipolaren Diathermie-Instrumentes überhaupt fliessen kann, braucht es eine ionisierte Lösung, wie z.B. Healon.

Wenn in weiterer Ausgestaltung der Erfindung die Impulswiederholungszeit Tw ist, kann die Impulsdauer Td zwischen Td min = (1/p)•Tw und Td max = (1/q)•Tw variiert werden, wobei vorzugsweise die Parameter p und q zumindest angenähert gleich 11 bzw. 3 sein können. Die entsprechend entstandene Pause wäre dann zumindest angenähert Tp max = Tw - Td min bzw. Tp min = Tw - Td max. Gute Resultate konnten auch mit Tw = 66 ms, Td min = 6 ms und Td max = 22 ms bzw. mit Tw = 11 ms, Td min = 1 ms und Td max = ca. 4 ms erreicht werden. Die angegebenen Werte für die Impulswiederholungszeit Tw, die lediglich als Beispiele dienen sollen, stellen selbstverständlich keine absoluten Grenzwerte dar. Vorzugsweise ist jedoch eine Impulswiederholungszeit Tw von mindestens 10 ms vorgesehen.

**Patentansprüche**

1. Kapsulotomie-Vorrichtung für die Augenmikrochirurgie mit einem Hochfrequenzgenerator, dessen Ausgang mit Modulatormitteln verbunden ist, um ein von diesem Hochfrequenzgenerator (30) erzeugtes Hochfrequenzsignal zu modulieren und mit einem Handstück mit einem bipolaren Diathermie-Instrument, dadurch gekennzeichnet, dass, um das Gewebe des Augenlinsensackes (12) zu durchtrennen, diese Modulatormittel (31,35) ausgestaltet sind, um periodisch das Hochfrequenzsignal gemäss einem Arbeitszyklus mit einer Impulswiederholungszeit Tw durchzulassen oder zu sperren, die eine Durchlasszeit $T_d$ und eine Sperrzeit $T_p$ gemäss den Bedingungen

    $T_p / T_d \geqq 2$
    $T_p + T_d \geqq 10$ ms

    mit einer Sperrzeit Tp von höchstens 60 Millisekunden und einer Durchlasszeit Td von mindestens 1 Millisekunde auf weist, dass die Modulatormittel (31,35) über einen Verstärker (40) mit der Primärwicklung eines Transformators (46) verbunden sind, und dass der Eingang (48,49) des Handstücks (21) über Leitungen an die Sekundärwicklung dieses Transformators (46) angeschlossen wird.

2. Vorrichtung nach Anspruch 1, gekennzeichnet durch die zusätzliche Bedingung $T_p / T_d \leqq 10$.

3. Vorrichtung nach Anspruch 1 oder 2, gekennzeichnet durch eine Durchlasszeit $T_d$ von mindestens 3 ms.

4. Vorrichtung nach einem der Ansprüche 1 bis 3, dadurch gekennzeichnet, dass für die maximale Sperrzeit $T_{pmax}$ die Beziehung 30 ms $\leqq T_{pmax} \leqq 60$ ms gilt.

5. Vorrichtung nach einem der Ansprüche 1 bis 4, dadurch gekennzeichnet, dass die Modulatormittel (31,35) ein UND-Tor (35) umfassen, dessen erster Eingang mit dem Ausgang des Hochfrequenzgenerators (30) und dessen zweiter Eingang mit dem Ausgang (34) einer Pulsbreitensteuerung (31) verbunden ist, um periodisch das UND-Tor gemäss dem besagten Arbeitszyklus zu öffnen oder zu sperren, und dass dieses UND-Tor (35) über den Verstärker (40) mit der Primärwicklung des Transformators (46) verbunden ist.

6. Vorrichtung nach einem der Ansprüche 1 bis 5, dadurch gekennzeichnet, dass der Verstärker (40) durch eine stabilisierte Spannungsquelle (42) mit einer Kurzschlussüberwachung gespeist wird.

7. Vorrichtung nach einem der Ansprüche 1 bis 6, dadurch gekennzeichnet, dass der Hochfrequenzgenerator (30) ein Signal mit einer Frequenz von zumindest angenähert 500 kHz erzeugt.

8. Vorrichtung nach einem der Ansprüche 1 bis 7, dadurch gekennzeichnet, dass der Verstärker ein Ausgangssignal mit einer Leistung bis Pmax = 54 W liefern kann.

9. Vorrichtung nach einem der Ansprüche 3 bis 8, dadurch gekennzeichnet, dass der Arbeitszyklus durch ein einstellbares frequenzkonstantes Rechtecksignal definiert ist, das eine Frequenz im Bereich zwischen 15 Hz und 30 Hz aufweist.

10. Vorrichtung nach einem der Ansprüche 3 bis 8, dadurch gekennzeichnet, dass die Impulswiederholungszeit 33 ms beträgt, und dass bei einer Impulsbreitenvariation zwischen 3 ms und 11 ms die entsprechend entstandene Pause 30 ms bzw. 22 ms lang ist.

**Claims**

1. Capsulotomy device for ocular microsurgery, having a high-frequency generator, whose output is connected to modulator means in order to modulate a high-frequency signal generated by the high-frequency generator (30) and having a handle comprising a bipolar diathermic instrument, characterised in that in order to pierce the tissue of the eye lens sac (12), these modulator means (31, 35) are so formed as periodically to transmit or block the high-frequency signal according to an operating cycle with a pulse repeat time Tw, having a transmission time Td and a blocking time Tp in accordance with the conditions:

    Tp / Td $\geqq$ 2
    Tp + Td $\geqq$ 10 ms,

    the blocking time Tp being at most 60 milliseconds and the transmission time Td at least 1 millisecond, in that the modulator means (31, 35) are connected to the primary winding of a transformer (46) via an amplifier (40), and in that the input (48, 49) of the handle (21) is connected via leads to the secondary winding of this transformer (46).

**2.** Device according to claim 1, characterised by the additional condition Tp / Td ≤ 10.

**3.** Device according to claim 1 or 2, characterised by a transmission time Td of at least 3 ms.

**4.** Device according to one of claims 1 to 3, characterised in that for the maximum blocking time Tp max, the equation 30 ms ≤ Tp max≤ 60 ms applies.

**5.** Device according to one of claims 1 to 4, characterised in that the modulator means (31, 35) comprise an AND-gate (35), whose first input is connected to the output of the high-frequency generator (30) and whose second input is connected to the output (34) of a pulse duration control (31) in order periodically to open or close the AND-gate according to said operating cycle, and in that this AND-gate (35) is connected via the amplifier (40) to the primary winding of the transformer (46).

**6.** Device according to one of claims 1 to 5, characterised in that the amplifier (40) is supplied with power from a stabilised voltage source (42) with a short-circuit monitoring device.

**7.** Device according to one of claims 1 to 6, characterised in that the high-frequency generator (30) generates a signal with a frequency of at least approximately 500 kHz.

**8.** Device according to one of claims 1 to 7, characterised in that the amplifier can supply an output signal with a power of up to $P_{max}$ = 54 W.

**9.** Device according to one of claims 3 to 8, characterised in that the operating cycle is defined by an adjustable, constant-frequency square-wave signal, which has a frequency in the range between 15 Hz and 30 Hz.

**10.** Device according to one of claims 3 to 8, characterised in that the pulse repeat time is 33 ms, and in that when the pulse duration is varied between 3 ms and 11 ms, the consequent pause is 30 ms or 22 ms long respectively.

**Revendications**

**1.** Dispositif de capsulotomie destiné à la microchirurgie oculaire, comportant un générateur haute fréquence, dont la sortie est raccordée à un dispositif de modulation, pour moduler un signal haute fréquence produit par ce générateur haute fréquence (30) et comportant une poignée dotée d'un instrument de diathermie bipolaire, caractérise en ce que pour percer le tissu de la poche du cristallin (12), ce dispositif de modulation (31,35) est conçu pour laisser passer ou pour arrêter périodiquement le signal haute fréquence selon un cycle de travail avec un temps de répétition d'impulsion Tw, qui présente un temps de passage $T_d$ et un temps d'arrêt $T_p$ selon les conditions

$$T_p / T_d \geq 2$$
$$T_p + T_d \geq 10 \text{ ms}$$

avec un temps d'arrêt $T_p$ de 60 millisecondes maximum et un temps de passage $T_d$ d'au moins 1 milliseconde, en ce que le dispositif de modulation (31,35) est raccordé par un amplificateur (40) au bobinage primaire d'un transformateur (46) et en ce que l'entrée (48,49) de la poignée (21) est raccordée par les conduites au bobinage secondaire de ce transformateur (46).

**2.** Dispositif selon la revendication 1, caractérisé par la condition supplémentaire $T_p / T_d \leq 10$.

**3.** Dispositif selon la revendication 1 ou 2, caractérisé en ce qu'un temps de passage $T_d$ est d'au moins 3 ms.

**4.** Dispositif selon l'une des revendications 1 à 3, caractérisé en ce que pour le temps d'arrêt maximum $T_{pmax}$, s'applique la relation 30 ms ≤ $T_{pmax}$ ≤ 60 ms.

**5.** Dispositif selon l'une des revendications 1 à 4, caractérisé en ce que le dispositif de modulation (31,35) comporte une porte ET (35) dont la première entrée est raccordée à la sortie du générateur haute fréquence (30) et dont la seconde entrée est raccordée à la sortie (34) d'une commande de largeur d'impulsion (31), pour ouvrir ou pour bloquer périodiquement la porte ET selon le cycle de travail précité et en ce que cette porte ET (35) est raccordée par l'intermédiaire de l'amplificateur (40) au bobinage primaire du transformateur (46).

**6.** Dispositif selon l'une des revendications 1 à 5, caractérisé en ce que l'amplificateur (40) est alimenté par une source de tension stabilisée (42) avec un contrôle de court-circuit.

**7.** Dispositif selon l'une des revendications 1 à 6, caractérisé en ce que le générateur haute fréquence (30) produit un signal d'une fréquence d'au moins approximativement 500 kHz.

8. Dispositif selon l'une des revendications 1 à 7, caractérisé en ce que l'amplificateur peut fournir un signal de sortie d'une puissance jusqu'à Pmax = 54 W.

9. Dispositif selon l'une des revendications 3 à 8, caractérisé en ce que le cycle de travail est défini par un signal rectangulaire de fréquence constante réglable qui comporte une fréquence dans la plage entre 15 Hz et 30 Hz.

10. Dispositif selon l'une des revendications 3 à 8, caractérisé en ce que le temps de répétition d'impulsion est de 33 millisecondes et en ce que pour une variation de largeur d'impulsion entre 3 millisecondes et 11 millisecondes, l'interruption produite est d'une durée de 30 millisecondes ou de 22 millisecondes.

Fig. 1

Fig. 2

# Fig. 3

# Fig. 4

3ms

30 ms Pause

11ms

22 ms Pause